# EUROPEAN PATENT APPLICATION

(11) **EP 1 671 666 A1**
(43) Date of publication of application: **21.06.2006**
(21) Application number: 04030158.2
(22) Date of filing: 20.12.2004
(51) Int. Cl.: A61M 5/168, A61B 19/00

(54) **Apparatus for applying a solution to a blood or lymph vessel and use thereof**

(71) Applicant: Reusch, Karl-Dieter, 50677 Köln (DE)
(72) Inventor: Reusch, Karl-Dieter, 50677 Köln (DE)
(74) Representative: Teipel, Stephan

(57) **Abstract**

The present invention relates to an apparatus (1,2,3,4) for applying a solution to a blood or lymph vessel, especially blood or lymph vessel transplants, and a method for testing a vessel or a method for maintenance and storage thereof using said apparatus is also disclosed.

## Description

The present invention relates to an apparatus for applying a solution to a blood or lymph vessel, especially blood or lymph vessel transplants, and a method for testing a vessel or a method for maintenance and storage thereof using said apparatus.

### Background of the invention

Coronary heart disease is one of the most important causes of death in western industrialized countries. The development of bypass surgery, during which a typically autologous vein graft is implanted to "bypass" the occluded heart vessel, was a milestone in the therapy of this disease.

The major short-coming of this therapy is re-occlusion of the graft vessel used as the bypass. After one year, re-occlusion occurs in about 15%, and after ten years in about 40% of the vein grafts with occlusion rates of 1 - 2% per year from the second to the sixth year and 4% here after. A similar situation is found in patients with peripheral arterial disease after surgical revascularization by an aorto-femoral bypass. After 5 years, the re-occlusion rates amount to 20-30%.

Although several patient related risk factors have been identified, e.g. morphological factors, gender, cigarette smoking, hyperlipidemia, hypertension, diabetes mellitus, and others, a second approach to prevent re-occlusion is directed towards the graft and its handling during recovery and maintenance before transplantation into the receiving site. Current harvesting procedures for e.g. vein grafts include the excision of the graft vessel from the donor site and in that the severing of several smaller branches from the graft vessel. Thereby several "leaks" are created, which need to be sutured or ligated before transplantation of the graft as bypass.

To make sure that all "leaks" have been closed and no open branches remain, the vessel must be tested. In order to identify such branches or leaks the surgeon typically enters the lumen of the vein graft at its first end with a cannula or syringe, occludes the second end with a clip and applies a physiologically acceptable solution such as heparinized saline solution via the cannula or syringe by manually operating the same. Through the syringe the solution is introduced in to the vessel lumen with a certain pressure, and exits through any leaks or branches due to the applied pressure. The applied pressures can be as high as 400 mmHg. The entire procedure is totally uncontrolled, since the applied pressure is exerted manually and is not measured.

After detection any remaining branches or leaks are ligated and the vessel is placed in a storage solution until it is implanted and continuous flow of blood or lymph is established through the bypass. The vessel needs, however, also to be irrigated after the distal anastomosis has been made to the heart or the peripheral organ. Some efforts to prevent vessel damage have been directed to the type of solution used for testing and storage. These are the so-called cardioplebic solutions. This approach has, however, proven as insufficient.

The pathophysiology of re-occlusion of a vein graft following aortocoronary bypass placement depends on the temporal sequence of pathophysiological events after intervention. The pathogenesis of saphenous vein graft disease can be described by three major phenomena: thrombosis, intima hyperplasia, and accelerated artherosclerosis. In any case, it has been found that the endothelium plays a crucial role in the processes involved.

Specifically, it has been shown that the pressure distension of blood vessels beyond 300 mmHg stimulates the expression of endothelial adhesion molecules in human vein grafts. In general, blood pressures largely exceeding physiological pressures in artherial or vein endothelial walls lead to direct mechanical destruction of the endothelium, and excessively high pressure even destroys smooth muscle cells. Steady low shear stress (2 dyne/cm²) likewise seems to be a biomechanical risk factor, because it induces a sustained and increasing release of endothelin-1, an increase in NfkappaB, MCP-1 and VCAM-1 expression and monocyte adhesion.

Vein grafts can, however, adapt to the non-physiological situation of artherial blood flow and pressure, which first causes proliferation of endothelial cells and thereafter proliferation of smooth muscle cells. Physiological conditions include a steady shear stress of about 16 dyne/cm² that approximates the average shear in the vasculature in vivo. Steady shear stress on a physiological level (16 dyne/cm²) induces a sustained low rate of NO in endothelial cells that protects the endothelium from inflammation and inhibits endothelial cell proliferation and atherogenesis.

It is finally known that the flow profile, i.e. the change of flow with time, forms another part of the shear stress experienced by a vessel. Experimental flow profiles include a ramp flow (slowly increasing flow, followed by a constant flow level), a step flow (rapid /abrupt increase followed by a constant flow level) or an impulse flow (rapidly fluctuating flow). A step flow leads to moderate endothelial cell proliferation and inflammation, whereas an impulse flow with a sharp increase and immediately subsequent sharp decrease results in strong endothelial cell proliferation and inflammation. A ramp flow followed by a steady flow level seems to have no negative effect on the function of endothelial cells. None of this knowledge has up to now been taken in consideration upon handling of graft vessels.

It is the object of the invention to provide an apparatus and a method which allows for reduced damaging of blood or lymph vessels especially upon testing and maintenance or storage during transplantation.

This object is achieved and other disadvantages of the prior art are overcome by the apparatus and the methods of the present invention. In a first aspect the invention thus provides an apparatus for applying a solution to a blood or lymph vessel, said apparatus comprising:
- a reservoir for a physiologically acceptable solution;
- a cannula for delivering the solution into the lumen of said vessel;
- means for controlling flow rate and/or pressure of the solution to prevent damage of the endothelial cells of said vessel;
- conduits connecting the reservoir, the means for controlling and the cannula, and
- optionally a sensor for measuring pressure and/or shear of the flowing solution.
   In a second aspect the invention provides a method for testing, maintenance and/or storage of a blood or lymph vessel, by
- providing an apparatus of the invention, and providing a physiologically acceptable solution within the reservoir,
- attaching the vessel at one end thereof to the cannula such that the vessel lumen and the lumen of the cannula are in flow communication, and
- flowing the physiologically acceptable solution from the reservoir through the cannula into the vessel lumen,
- wherein a flow rate and pressure of said solution are controlled to prevent damage of the endothelial cells of said vessel.

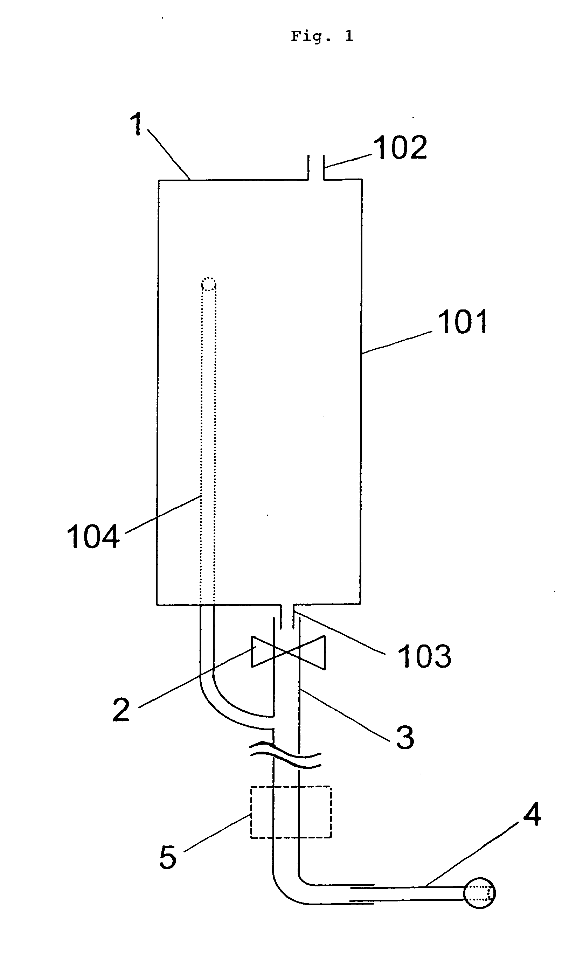

Fig. 1 shows a preferred embodiment of the apparatus according to the present invention. In this embodiment of the apparatus the solution flow is driven gravitationally and flow control is exerted by a manually operated valve.

The apparatus and methods of the invention are based on the finding that re-occlusion of vessels after transplantation thereof can be prevented or substantially reduced when controlling shear stress and/or pressure stress experienced by said vessels during handling of the same. Although the above information on endothelial cell susceptibility on shear stress or pressure and associated damages was known, this was constantly disregarded by surgeons in transplantation surgery. It is the merit of the present invention to notice importance of even short term shear stress, and to provide for a practical apparatus for handling i.e. testing, maintenance and storage of vessels, especially during transplantation surgery.

In the apparatus according to the invention the flow rate is preferably controlled to provide an initial flow rate ramp and a maximum flow rate such that, at the maximum flow rate, a shear stress of between 3 and 20 dyne/cm², preferably 14 to 16 dyne/cm² and most preferably about 16 dyne/cm² is exerted on and experienced by the vessel walls. Alternative, or preferably in addition, the flow rate is controlled such that the maximum pressure of the solution is in the range of 40 to 100 mmHg, preferably 45 to 55 mmHg and most preferably about 50 mmHg, when measured within the vessel lumen or a conduit of the apparatus in direct flow communication therewith.

The initial flow rate ramp typically provides for an increase in flow of solution and hence an increase in shear and pressure over a time period of 1 sec. to 10 min., preferably 3 sec. to 1 min., and most preferably of 5 sec. to 30 sec. In this time period the flow increases to the maximum desired shear and/or pressure value as indicated above.

The means for controlling said flow rate can generally be any means suitable for said purpose such as a pump, a clip, a stop cock, a valve or similar. Preferably the apparatus also comprises a sensor for measuring pressure of the flowing solution and/or shear exerted by the flowing solution. Any suitable shear or pressure sensor can be used. The sensor is placed downstream of the means for controlling flow in the conduit connecting said means with said cannula. Flow conditions in said conduit are assumed to resemble those within the vessel.

In a preferred embodiment, the apparatus includes a sensor and a valve or stop cock, which is optionally operated by said sensor. In a preferred embodiment the apparatus includes a graduated cylinder with top inlet and bottom outlet, a conduit attached to said outlet, a valve or stop cock within said conduit, and an internal ascending pipe within the cylinder, which is connected to the conduit downstream said valve or stop cock.

The means for controlling the flow can in parallel be the means driving said flow as in case of a pump. Alternatively or in addition thereto, the flow can be gravitationally driven such as by use of a conventional infusion system including a bag or other reservoir, catheter and needle, where flow is caused by gravitational forces.

In a preferred embodiment the apparatus of the invention includes a valve or valve system for exerting flow control. The valve or valve system can be operated manually or in semi- or fully automated fashion. Any suitable valve or valve system known in the art can be used. Manual operation is preferred in some embodiments, especially in combination with gravitationally driven systems in view of simplicity of the apparatus and costs. In case of semi- or fully automated operation the valve or valve system may be operated or adjusted via a feedback loop, based on the measurement results received from a sensor downstream as described above.

The means for controlling may be provided independently or integrated into the reservoir for the physiologically acceptable solution. In one embodiment it is preferably integrated into the reservoir e.g. in form of a flow control valve at the reservoir outlet. Alternatively, it can be provided in separate manner, as e.g. in case of a peristaltic or rolling pump.

In a preferred embodiment the apparatus according to the present invention further includes a data processing unit operatively associated with said means for controlling and optionally with said sensor, if present. It may further include a data input port, a data storage unit and a data output port.

In any case, the apparatus includes conduits, connecting in flow communication the reservoir, the means for controlling flow and the cannula lumen. Suitable conduits are known in the art, and need not be described here.

The cannula of the apparatus can be any suitable cannula, provided it allows delivery of the solution into the vessel lumen. Suitable cannulae are known in the art. Preferably the cannula is a blunt ended cannula or a bulb head cannula (Knopfkanüle). In any case the cannula has appropriate internal and external diameters to allow for insertion into the vessel concerned and to allow for the desired solution flow, respectively.

The apparatus according to the present invention comprises at least one reservoir for the physiologically acceptable solution. The reservoir can be of any suitable form known in the art. Typically, it will be in the form of a bottle, cylinder or bag. The apparatus of the invention may further comprise an additional reservoir for said physiologically acceptable solution. For example in a preferred embodiment a graduated cylinder may form the first reservoir, which at its inlet end is connected to an upstream bottle, forming a second reservoir. The use of a second reservoir has the advantage that the second reservoir may be refilled or exchanged, when empty, without interrupting flow from the first reservoir. The second reservoir can be connected to the first reservoir by any appropriate means such as e.g. a Luer lock or similar.

The apparatus of the invention may further comprise one or more of the following: at least one additional valve, one or more connectors, refill openings e.g. in form of a luer lock, one or more clips for interrupting flow, a Luer connection to said cannula, and a holding device for the reservoir, e.g. for adjusting static height of the same. In any case the apparatus should allow for sterilization of the same and should be adapted to sterile delivery of the solution into the vessel lumen.

The above apparatus is suitable for testing, maintenance and for storage of a vessel especially during transplantation surgery. The invention thus also provides a method for testing a vessel and a method for maintenance or storage of said vessel. Both method make use of the apparatus as described above. The methods therefore comprise the steps of:
- providing the apparatus as described above and also providing a physiologically acceptable solution in the reservoir thereof,
- attaching the vessel at one end thereof to the cannula such that the vessel lumen and the lumen of the cannula are in flow communication, and
- flowing the physiologically acceptable solution from the reservoir through the cannula into the vessel lumen.

During said flow the flow rate and/or pressure of said solution are controlled to prevent damage of the endothelial cells of said vessel as described above. The solution may leave the vessel lumen at the second end opposite to the first end where the cannula is attached. Alternatively, the vessel may at its second end be attached to the body such as to the heart. In this case the physiologically acceptable solution is introduced into the patient's body. Thereby the method of the invention even allows for irrigating the vessel after anastomosis has been made.

In the method for testing, the vessel may also be closed at the vessel end opposite to the end the cannula is attached to. This can e.g. be achieved by a conventional clip. Upon initiating of solution flow, the solution will, due to the increasing pressure, exit the vessel through any existing leaks, thereby allowing their detection. Accordingly, the apparatus can be used for detecting leaks in the vessel or in other words testing of the vessel for existing leaks, if any. Flow of the solution is preferably discontinued, once the leaks have been detected.

Alternatively the second vessel end may be re-opened and the solution flow may be continued for maintenance of the vessel, preferably until natural liquid flow, such as blood flow through the vessel is re-established. The apparatus may even be used to constantly perfuse the vessel after the distal anastomosis has been made to the heart or the peripheral organ.

The vessel which can be tested or maintained by the apparatus and methods of the invention is preferably an artery, a vein or a lymph vessel. The vessel can be partially or fully detached from a body. The vessel preferably is a transplant, which can be natural or synthetic.

Preferably it is a natural vessel transplant, more preferably an autologous vessel transplant. In case of bypass surgery, it can be a vein graft retrieved from e.g. the leg of the patient.

The physiologically acceptable solution for use in the present invention can in principle be any physiologically acceptable, preferably pharmaceutically and physiologically acceptable solution. Suitable solutions are well known to the skilled worker and need not be detailed here. Preferably it is a cardioplebic solution, i.e. a solution intended to minimize damage to endothelial cells. Suitable solutions are for example a heparinized saline solution and PBS.

The present invention will in the following be further illustrated by describing a preferred embodiment thereof. Referring to Fig. 1, the apparatus of the invention comprises a reservoir for a physiologically acceptable solution (1) which is provided in form of a graduated cylinder (101) with top inlet (102) and bottom cylinder outlet (103) in flow communication with conduit (3). Conduit (3) connects the cylinder outlet (103) and valve (2). Valve (2) allows for control of fluid flow through conduit (3). The top inlet may be provided with a luer lock for easy refill. The graduated cylinder further comprises an ascending pipe (104), connected to conduit (3) downstream said valve (2). The valve (2) may be exchanged for a stop cock.

The graduated cylinder (101) is filled with a physiologically acceptable solution and placed above the vessel or surgery table, e.g. by means of corresponding holders (not shown). The height of the liquid level above the vessel determines the liquid flow velocity through the cylinder outlet and pressure in any downstream conduit. Maximum shear is further determined by the conduit diameter. The opening width of valve (3), which in this case is a manually operated valve, determines actual shear and pressure.

When passing the connection of ascending pipe (104) the flowing solution depending on its pressure or shear changes the solution level in the ascending pipe. The applied pressure or shear can thus be derived from the height of the liquid meniscus in the ascending pipe, which has been gauged depending on the conduit diameter and the type of solution beforehand. Valve (2) forms the means for flow control in conduit (104). Manual opening (or closing) of the valve increases (or decreases) flow of the solution. The resulting shear or pressure can be read out from the ascending pipe, and the opening of the valve can be adjusted accordingly.

Conduit (3) connects to cannula (4) at the proximal end thereof. Cannula (4) is a bulb head cannula, having rounded part at its distal end. Thereby damages to the vessel can be prevented upon insertion of the cannula into the vessel lumen.

Alternative or in addition to the ascending pipe, another pressure sensor (5) such as an electronic pressure sensor can be installed downstream of valve (2). If desired, the pressure sensor may send data to a data processing unit (not shown). Data processing unit may then be designed to operate valve (2) or to provide a preferably visible (e.g. digital) data output, according to which the valve (2) is again adjusted manually.

The above description of a preferred embodiment is not intended to limit the present invention, which shall be defined in the appending claims.

## Claims

1. Apparatus for applying a solution to a blood or lymph vessel, said apparatus comprising:
- a reservoir for a physiologically acceptable solution;
- a cannula for delivering the solution into the lumen of said vessel;
- means for controlling flow rate and/or pressure of the solution to prevent damage of the endothelial cells of said vessel; and
- conduits connecting the reservoir, the means for controlling and the cannula.

2. The apparatus of claim 1, wherein the flow rate is controlled to provide an initial flow rate ramp and a maximum flow rate such that a shear stress of between 3 and 20 dyne/cm², preferably 14 to 16 dyne/cm² and most preferably about 16 dyne/cm² is exerted on the vessel walls.

3. The apparatus of claim 1 or 2, wherein the flow rate is controlled such that the maximum pressure of the solution is in the range of 40 to 100 mmHg, preferably 45 to 55 mmHg and most preferably about 50 mmHg.

4. The apparatus of one of the preceding claims further comprising a sensor for measuring pressure and/or shear of the flowing solution.

5. The apparatus according to one of the preceding claims, wherein the means for controlling flow rate is selected from the group consisting of a valve, a clip, a stop cock and a pump.

6. The apparatus according to claim 4 and 5, including a sensor and a valve.

7. The apparatus of claim 6 wherein the apparatus includes a graduated cylinder with top inlet and bottom outlet, a conduit attached to the bottom outlet, a valve or stop cock within said conduit, and an internal ascending pipe within the cylinder, which is connected to the conduit downstream said valve or stop cock.

8. The apparatus of claim 5, 6 or 7, wherein the valve is operated manually.

9. The apparatus of one of the preceding claims, wherein the means for controlling is integrated into the reservoir.

10. The apparatus according to one of the preceding claims, wherein the cannula is a blunt ended cannula or a bulb head cannula.

11. The apparatus according to one of the preceding claims, further comprising an additional reservoir for said physiologically acceptable solution.

12. The apparatus according to one of the preceding claims, further comprising one or more of the following: at least one additional valve, one or more clips for interrupting flow through at least one of the conduits, one or more connectors, and a holding device.

13. A method for testing of a blood or lymph vessel, said method comprising the steps of:
- providing the apparatus according to one of the preceding claims and providing a physiologically acceptable solution within the reservoir;
- attaching the vessel at one end thereof to the cannula such that the vessel lumen and the lumen of the cannula are in flow communication, and
- flowing the physiologically acceptable solution from the reservoir through the cannula into the vessel lumen,
- wherein a flow rate and pressure of said solution are controlled to prevent damage of the endothelial cells of said vessel.

14. A method for maintaining and/or storing a lymph or blood vessel, said method comprising the steps of:
- providing the apparatus according to one of the preceding claims and providing a physiologically acceptable solution within the reservoir;
- attaching the vessel at one end thereof to the cannula such that the vessel lumen and the lumen of the cannula are in flow communication, and
- flowing the physiologically acceptable solution from the reservoir through the cannula into the vessel lumen,
- wherein a flow rate and pressure of said solution are controlled to prevent damage of the endothelial cells of said vessel.

15. The method according to claim 13 or 14, wherein the flow rate is controlled to provide an initial flow rate ramp and a maximum flow rate such that a shear stress of between 3 and 20 dyne/cm², preferably 14 to 16 dyne/cm² and most preferably about 16 dyne/cm² is exerted on the vessel walls.

16. The method according to one of claims 13 to 15, wherein the flow rate is controlled such that the maximum pressure of the solution is in the range of 40 to 100 mmHg, preferably 45 to 55 mmHg and most preferably about 50 mmHg.

17. The method according to one of claims 13, 15 or 16, wherein the vessel is closed at the vessel end opposite to the end the cannula is attached to.

18. The method according to one of claims 13 to 17, wherein the vessel is an artery, a vein or a lymph vessel.

19. The method according to one of claim 13 to 18, wherein the physiologically acceptable solution is a cardioplebic solution.
